# EUROPEAN PATENT SPECIFICATION

(11) **EP 0 152 274 B1**
(45) Date of publication and mention of the grant of the patent: **20.01.1993**
(21) Application number: 85300801.9
(22) Date of filing: 06.02.1985
(51) Int. Cl.: C12Q 1/37

(54) **Method for the determination of Leucine Aminopeptidase (LAP)**
Verfahren zur Bestimmung von Leucinaminopeptidase (LAP)
Méthode de détermination de Leucine Aminopeptidase (LAP)

(30) Priority: 07.02.1984 JP 20194/84
(43) Date of publication of application: 21.08.1985
(73) Proprietor: Kyowa Medex Co. Ltd., Tokyo (JP)
(72) Inventor: Miike, Akira, Sunto-gun Shizuoka-ken (JP); Katsumata, Yukio, Susono-shi Shizuoka-ken (JP); Tatano, Toshio, Numazu-shi Shizuoka-ken (JP)
(74) Representative: Lambert, Hugh Richmond

(56) References cited:
- EP-A- 0 103 823
- DE-A- 1 673 281
- DE-A- 1 931 057
- FR-A- 2 392 960
- FR-A- 2 509 324
- FR-A- 2 532 307
- GB-A- 1 176 968
- GB-A- 2 022 079
- US-A- 3 535 113
- US-A- 3 971 769
- US-A- 4 087 331
- CHEMICAL ABSTRACTS, vol. 96, no. 17, 26th April 1982, pages 401, abstract no. 138895j, Columbus, Ohio, US; & JP-A-81 148 299 (TOYOBO CO., LTD) 17-11-1981

## Description

The present invention relates to a method for the determination of the activity of the enzyme leucine aminopeptidase (hereinafter referred to as LAP) and a composition for use therein.

In the determination of LAP activity it is known to react the enzyme with a suitable substrate obtained by binding leucine to a chromogen, to form a colored compound which is then determined colorimetrically.

The chromogens proposed for this purpose include p-nitroanilide, β-naphtylamide, 4-hydroxy-3-carboxyanilide, p-hydroxyanilide, 4-N,N-dialkylamino-3-carboxyanilide and 4-N,N-dialkylaminoanilide.

However, the solubility of these compounds is, at most, 6.5 mg/ml, and it is this solubility that controls and limits the rate of reaction. This method of determination of LAP activity is therefore slow since a long period of time is required to complete the enzymatic reaction. Other disadvantages are that the results are influenced by other components in the sample, the reaction has to be carried out under strongly alkaline conditions and the sensitivity is low because of the low molecular extinction coefficient of the pigments formed. Thus altogether the accuracy of this method of determining LAP activity is low.

In GB-A-2 022 079 there are described novel L-leucyl-4-hydroxyanilide derivatives for use in the determination of LAP activity and which are alleged to be of high solubility and high reactivity and which are alleged to overcome the problems the then known chromogens for the determination of LAP activity, namely, safety, reproducibility, stability, reactivity and selectivity. The novel L-leucyl-4-hydroxyanilide derivatives described in GB-A-2 022 079 are of the formula 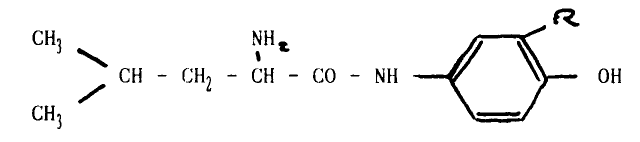
where R is carboxy or a sulfo-group. Of the two compounds described (R equals -COOH or -SO₃H) the highest substrate reactivity (263 units) is ascribed to the compound L-leucyl-3-sulfo-4-hydroxyanilide.

In accordance with the present invention, it has been found that good results are obtained using L-leucyl anilides of aniline derivatives having a sulfoalkyl-substituted amino group in the 4-position. Such aniline derivatives are known per se and have been proposed as diffusion-fast, colour-forming developing agents in photographic processing, see GB-A-1309 133. However, the L-leucyl anilides of those derivatives are not known, and it is those L-leucyl anilide derivatives that form the subject matter of the present invention both as novel compounds per se and as chromogenic substrates in the determination of LAP activity, and for which purpose they show excellent solubility and provide a pigment of high stability and with a high molecular extinction coefficient so that it is possible to carry out the colorimetric determination of the pigment in the visible region of the spectrum.

In accordance with one aspect therefore of the invention LAP activity is determined by reacting the enzyme with a substrate of the formula (I) 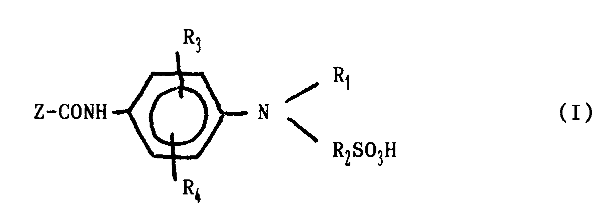
wherein Z represents (CH₃)₂CHCH₂CH(NH₂)-, R₁ represents hydrogen, alkyl or substituted alkyl, R₂ represents alkylene or hydroxyalkylene, R₃ and R₄ are the same or different and represent hydrogen, halogen, nitro, hydroxyl, sulfo, carboxyl, alkyl or alkoxy, or a salt thereof, thereby to produce a compound of the formula (II) 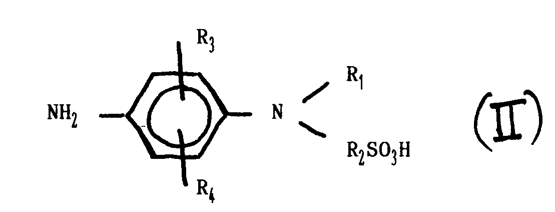
wherein R₁, R₂, R₃, and R₄ are as defined for formula I, and either reacting compound (II) with a chromogen to form a pigment, or converting compound (II) into diazonium salt and reacting the diazonium salt with coupling agent to form azo dye, and measuring the absorbency of the colored solution comprising said pigment or said azo dye.

In accordance with other aspect, the invention provides novel chromogenic substrates for the determination of LAP activity being the compounds of formula I above, and reagent solutions for the determination of LAP activity comprising such a substrate in combination with either a chromogen reactive with the product of the reaction of that substrate with LAP to produce a pigment, or with a diazotising reagent which is reactive therewith and then with a coupling agent to produce an azo dye.

In the definition of R₁ - R₄, alkyl includes alkyl having 1 - 5 carbon atoms such as methyl, ethyl, propyl, butyl and pentyl, halogen includes chlorine, bromine or iodine, the alkyl moiety in alkoxy has the same meaning as defined above for alkyl, alkylene includes alkylene having 1 - 5 carbon atoms such as methylene, ethylene, tetramethylene and pentamethylene, the alkylene moiety in hydroxyalkylene having the same meaning, and the possible substituents in substituted alkyl include sulfo, hydroxyaryl, nitro, carboxyl, and halogen such as chlorine or bromine.

The compounds (I) can be synthesized by the following method. 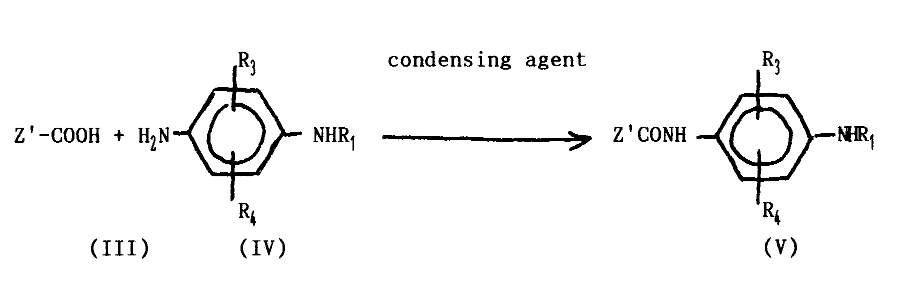 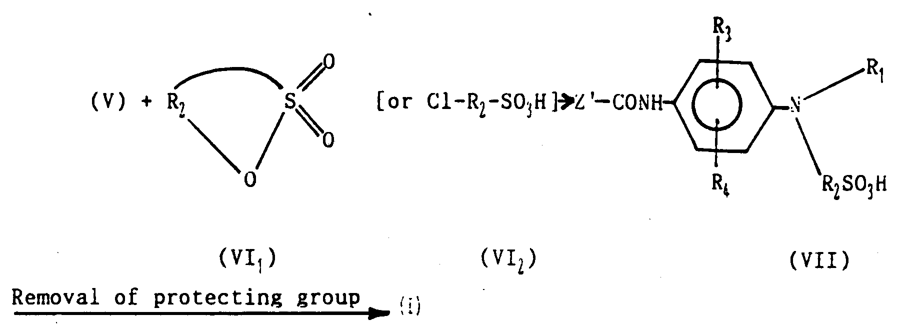
Where Z' is a group as defined for Z but in which the amino group is protected by an amino protecting group. Examples of suitable protecting groups include benzyloxycarbonyl and tertbutoxycarbonyl (BOC).

An example of compound (III) is N-BOC-L-leucine. As compound (IV), phenylenediamine or phenylenediamines having substituents corresponding to R₃ or R₄ of the desired compound may be used. A suitable condensing agent is, for example, DCC.

The first reaction is carried out at room temperature to 60⁰C for 1 to 10 hours in an organic solvent. To the reaction mixture as such, or compound (V) isolated therefrom, is added the Compound (VI). The second reaction is then carried out at room temperature to 60⁰C for 15-30 hours.

Suitable examples of Compound (VI) are shown in reference Example 2 described later. The desired compound, Compound I , is finally obtained by isolating compound (VII) after completion of the second reaction and removing the amino protecting group in a manner known per se.

Examples of suitable chromogens used to form the pigment by reaction with the compound (II) formed by the enzymatic reaction include amines such as N,N-diethyl-m-toluidine, N-ethyl-N-hydroxyethyl-m-toluidine, N-ethyl-N-3-methylphenyl-N'-acetylethylenediamine (EMAE), N-ethyl-N-(3-methylphenyl)-N'-succinylethylenediamine (EMSE), N,N-di-3-sulfopropyl-m-toluidine, N-ethyl-N-2-hydroxy-3-sulfopropyl-m-toluidine (TOOS), N,N-dimethyl-m-toluidine, N-ethyl-N-2-cyanoethyl-m-toluidine, N-(2-hydroxy-3-sulphopropyl)-m-toluidine, aniline derivatives, such as N-ethyl-N-(3-sulfopropyl)-3,5-dimethoxyaniline, N-ethyl-N-(2-hydroxy-3-sulfopropyl)-3,5-dimethoxyaniline, N-ethyl-N-(3-sulfopropyl)-3-methoxyaniline, N-ethyl-N-2-cyanoethyl-aniline, N,N-dimethylaniline and N,N-diethyl-aniline, phenols such as α-naphthol, β-naphthol, o- or p-bromophenol, o- or m-anisole, 2,6-xylenol, 3,5-xylenol, 2,5-xylenol, 2,3-xylenol and o- or m-cresol.

These chromogens are used in a concentration of 0.01 - 5 mg/ml with a surfactant such as Triton X-100, Brij-35 and Brij-80 in a concentration of 0.01 - 2% especially in the case of chromogens having low solubility.

Cinnamaldehydes such as p-N,N-dimethylamino cinnamaldehyde, p-N,N-diethylamino cinnamaldehyde may also be used as chromogens at a concentration of 0.01 - 100 mg/ml. In this case an azo dye is formed as the pigment.

In the alternative method of converting a compound (II) into diazonium salt, sodium, potassium or ammonium nitrite may be used at a concentration of 0.1 - 100 mg/ml, and as the final coupling agent, phenols such as phenol and 3,5-xylenol may be used in a concentration of 0.01 - 100 mg/ml.

When the formed pigment is not an azo dye, the reaction of compound (II) with a chromogen is usually carried out in the presence of an oxidizing agent or oxidase.

Examples of suitable oxidizing agents are inorganic compounds such as hydrogen peroxide, sodium or potassium metaperiodate, sodium or potassium permanganate, sodium or potassium dichromate, sodium or potassium matavanadate, pentacyanoiron complexes, and peroxy acids, such as peracetic acid. Suitable oxidases are hydrogen peroxide-peroxidase (HRP), and oxidases such as cerulopiasmin and laccase (E C. 1. 10. 3. 2.).

The inorganic oxidizing agent may be used in a concentration of 0.01 - 10 mg/ml and the oxidases at a concentration of 0.1 - 10 U/ml.

Tables 1 and 2 show the solubility, maximum absorption wavelength, sensitivity and stability of the formed pigments:

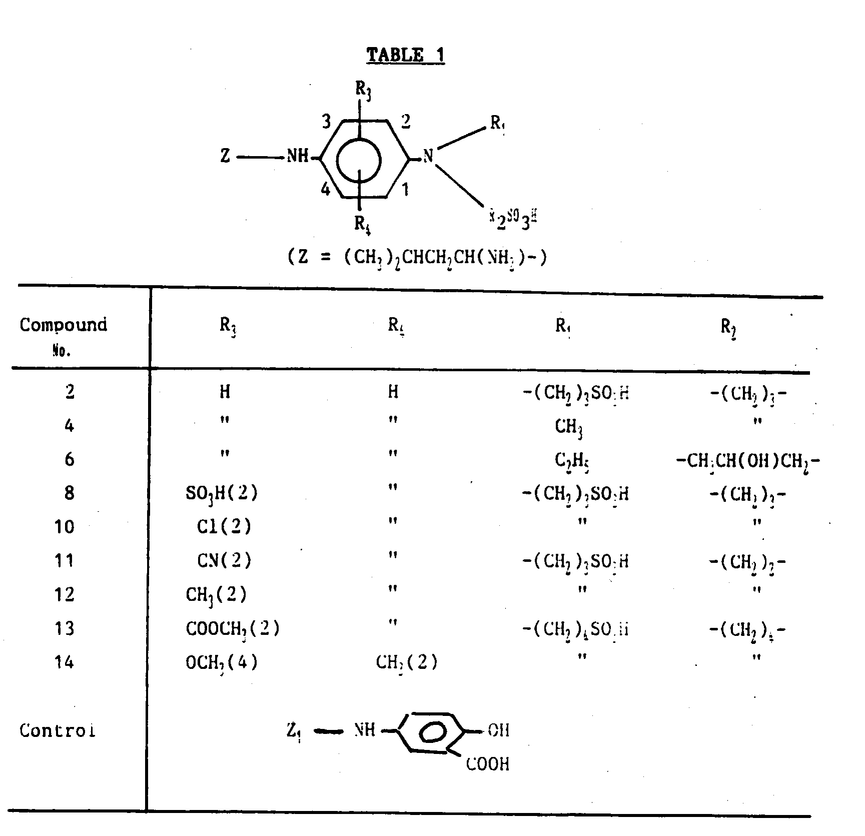

Numbers in parenthesis of R₃ or R₄ show the position of the group R₃ or R₄ on the benzene nucleus.

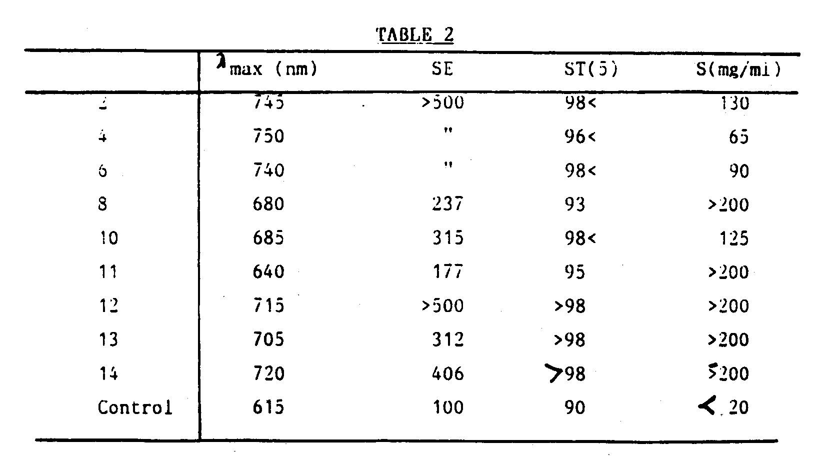

- ^{λ}max: : Maximum absorption wavelength
- SE: : Sensitivity of pigment
- ST: : Stability of pigment
- S: : Solubility

### Sensitivity:

50 µl of 1 mM each free compound was added to 3 ml of 0.1 M phosphate buffer containing 0.5 mg/ml EMSE and 1 U/ml laccase and the mixture was incubated at 37⁰C for 5 minutes. The absorption of the reaction solution at ^{λ}max was measured. As a control, 5-aminosalicylic acid was used and the reaction of the compound with 2,6-xylenol was carried out in the presence of metaperiodate under strong alkaline conditions.

The results are shown, defining the sensitivity of 5-aminosalicylic acid as 100.

The enzymatic reaction is usually carried out in a buffer solution having a pH of 6 to 10 preferably around 8. As the buffer, good buffer, phosphate buffer, acetate buffer, etc. may be used. The present method is very simple and excellent. For example, the solubility of the present substrate is very high and therefore it is not necessary to add surfactant or organic solvent to increase the solubility. The measurement of absorption is carried out at a wavelength of 560 nm or more and therefore the results are not influenced by other components present in the test serum. Since the sensitivity and stability of the present substrates are excellent, more accurate results are obtainable.

Another aspect of the present invention resides in a test composition for the determination of LAP activity which comprises a compound of formula (I) and a chromogen. The composition may also contain an oxidizing agent, an oxidase, a diazotizing agent, a coupling agent or a buffer.

Certain specific embodiments of the invention are illustrated by the following representative examples.

### Example 1

| Reagent solution (pH 7.3) | |
|---|---|
| Compound No. 2 | 0.03 g/ml |
| EMSE | 0.5 mg/ml |
| Magnesium nitrate | 1 mg/ml |
| Good buffer | 8 mg/ml |
| Laccase | 1 U/ml |

3 ml of the reagent solution was incubated at 37°C for ten minutes. To the solution was added 0.05 ml of serum sample and the mixture was immediately stirred and the change in absorption of the solution at 745 nm was measured for one minute.

LAP activity in the sample can be calculated using the following equation.

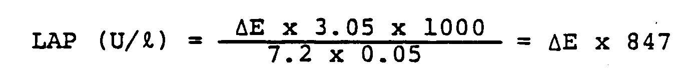

ΔE means the amount of absorption change per minute.

The same procedures as described above were repeated except that the following materials were used.
(a)
   - Substrate: : L-leucyl-3-carboxy-4-hydroxyanilide
   - Oxidizing agent: : sodium metaperiodate
   - Coupling agent: : p-xylenol
   - Wavelength for measurement: : 635 nm
(b)
   - Substrate: : L-leucyl-p-nitroanilide
   - Coupling agent: : p-dimethylaminocinnamaldehyde
   - Wavelength for measurement: : 565 nm
(c)
   - Substrate: : L-leucyl-p-N,N-diethylaminoanilide
   - Oxidizing agent: : m-periodate
   - Coupling agent: : 1-naphtol-2-sulfonic acid
   - Wavelength for measurement: : 675 nm
The amounts of absorption change per one minute were measured for ten samples and the results are shown in Table 3.

**Table 3**

| | (a) | (b) | (c) | The method according to the present invention |
|---|---|---|---|---|
| Serum(1) | 0.0188 | 0.0071 | 0.0158 | 0.0454 |
| (2) | 0.0127 | 0.0054 | 0.0095 | 0.0395 |
| (3) | 0.0198 | 0.0090 | 0.0150 | 0.0543 |
| (4) | 0.0203 | 0.0089 | 0.0145 | 0.0541 |
| (5) | 0.0148 | 0.0071 | 0.0116 | 0.0382 |
| (6) | 0.0158 | 0.0071 | 0.0126 | 0.0437 |
| (7) | 0.0153 | 0.0074 | 0.0113 | 0.0400 |
| (8) | 0.0191 | 0.0086 | 0.0150 | 0.0532 |
| (9) | 0.0190 | 0.0082 | 0.0138 | 0.0537 |
| (10) | 0.01840 | 0.0079 | 0.0133 | 0.0476 |

### Example 2

The reagent solution wherein laccase was excluded from the reagent solution of Example 1 was used.

3 ml of the reagent solution was incubated at 37°C for 10 minutes and to the mixture was added 0.05 ml of serum sample. The mixture was immediately stirred and incubated at 37°C for 10 minutes. After ten minutes passed,
(a) 0.03 ml of 10 mg/ml α-naphtol methanol solution and 0.1 ml of 1N-NaOH solution containing 50 mg/ml of sodium meta-periodate were added to the solution. The mixture was incubated at 37°C for 10 minutes and the absorption of the reaction solution at 600 nm was measured.
(b) 1 ml of 10 mg/ml p-N,N-dimethylamino cinnamaldehyde ethanol solution and 1 ml of 0.4N-HCl solution were added to the solution and the mixture was incubated at 37°C for 10 minutes. The absorption of the reaction solution at 570 nm was measured.
(c) 0.5 ml of 1N-HCl and 0.5 ml of 1 mg/ml sodium nitrite were added to the solution and the mixture was incubated at 37°C for 10 minutes. Then 0.1 ml of 1 mg/ml 3,5-xylenol ethanol solution was added thereto and the absorption of the reaction solution at 650 nm was measured.

The results are shown in Table 4

**Table 4**

| | (a) | (b) | (c) | Example 1 |
|---|---|---|---|---|
| Serum 1 | 0.331 | 0.386 | 0.277 | 0.0454 |
| 2 | 0.291 | 0.336 | 0.241 | 0.0395 |
| 3 | 0.389 | 0.466 | 0.330 | 0.0543 |
| 4 | 0.395 | 0.460 | 0.325 | 0.0541 |
| 5 | 0.277 | 0.320 | 0.230 | 0.0382 |
| 6 | 0.322 | 0.372 | 0.270 | 0.0437 |
| 7 | 0.291 | 0.333 | 0.244 | 0.0400 |
| 8 | 0.386 | 0.453 | 0.325 | 0.0532 |
| 9 | 0.392 | 0.460 | 0.331 | 0.0537 |
| 10 | 0.346 | 0.405 | 0.292 | 0.0476 |
| Ratio of sensitivity | 73 | 85 | 61 | 100 |

### Reference Example 1

Production of hydrobromide of L-leucyl-p-N,N-disulfopropyl aminoanilide.

2.49 g of N-BOC-L-leucine (hereinafter referred to as BLL) was dissolved in 100 ml of dioxane. To the solution were added 4.12 g of DCC and 2.16 g of p-phenylene-diamine (hereinafter referred to as PDD), and the mixture was incubated at room temperature for 4 hours. The mixture was filtered and the filtrate was concentrated to dryness under reduced pressure. The product was redissolved in methanol/water = 80/20 (vol/vol) and the solution was subjected to chromatography using Diaion HP-20 (Produced by Mitsubishi Chemical Industries Ltd.) and methanol/water = 80/20 was used as eluting agent.

Methanol was removed from the eluate under reduced pressure to obtain N-BOC-L-leucyl-p-aminoanilide in the yield of 1.25 g. 1 g of the product was dissolved in 50 ml of chloroform. To the mixture were added 2 ml of triethylamine and 5 g of 1,3-propanesultone and the mixture was incubated at 50° for one day. 50 ml of ethylether was added to the mixture which was separated into two layers. To the underlayer were added 5 ml of acetic acid and 20 ml of 25% hydrogen bromide-acetic acid to remove BOC group and the mixture was allowed to stand at room temperature. To the mixture was added 50 ml of ethylether to form L-leucyl-p-N,N-disulfopropyl aminoanilide · HBr as a precipitate. After filtration with glass filter, the precipitate was dissolved in 50 ml of methanol and the solution was neutralized with 6N-NaOH. After removal of formed NaCl by filtration, ethylether was added to form a precipitate. The precipitate was separated by filtration to obtain 0.63 g of desired compound having a melting point of 221 -223°C

| Elemental analysis | | | |
|---|---|---|---|
| | C | H | N |
| Found (%) | 46.29 | 6.58 | 9.07 |
| Calculated (%) | 46.45 | 6.67 | 9.03 |

## Claims

1. A method for the determination of leucine-aminopeptidase (LAP) activity in a sample which comprises reacting the enzyme with an enzyme substrate to produce a product compound determinable by means of a colorimetric reaction, characterised by the steps of: (a) reacting the enzyme with an enzyme substrate of the formula (I): 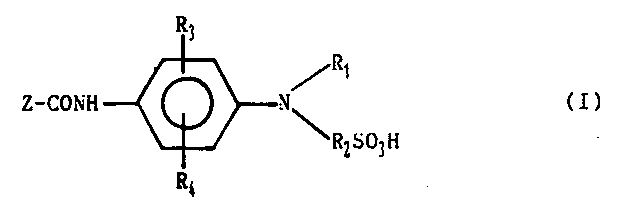 wherein Z represents (CH₃)₂CHCH₂CH(NH₂)-, R₁ represents hydrogen, alkyl or substituted alkyl R₂ represents alkylene or hydroxyalkylene, R₃ and R₄ are the same or different and represent hydrogen, halogen, nitro, hydroxyl, sulfo, carboxyl, alkyl and alkoxy, or a salt thereof, thereby to produce a product compound of the formula (II): 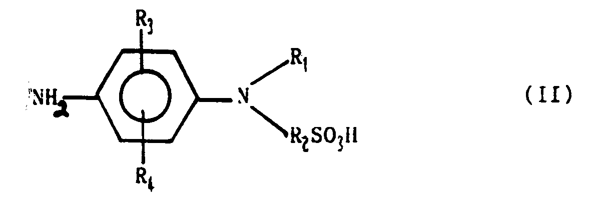 wherein R₁, R₂, R₃, and R₄ are as defined above; (b) reacting the compound of formula (II) with a chromogen to form a pigment or azo dye or converting the compound of formula (II) into diazonium salt and reacting the diazonium salt with coupling agent to form an azo dye; and (c) measuring the absorbtion of the colored reaction solution.

2. A method according to claim 1, wherein in step (b), the reaction of compound (II) with the chromogen is carried out in the presence of an oxidizing agent, that reaction producing a coloured product which is other than an azo dye.

3. A method according to claim 2, wherein said oxidizing agent is an oxidase.

4. A method according to claim 1, 2 or 3, wherein, in the formulae (I) and (II), R₁ is sulfoalkyl.

5. A method according to claim 1, 2 or 3, wherein the enzyme substrate used in step (a) is L-leucyl-p-N,N-disulfopropylamino anilide.

6. A method according to any one of claims 1 to 5, wherein, in step (b), the compound of formula (II) is reacted with an amine, aniline derivative or a phenol as said chromogen.

7. A method according to claim 6, wherein the chromogen is EMSE.

8. A method according to claim 1, 4 or 5, wherein, in step (b), the compound of formula (II) is reacted with cinnamaldehyde to produce an azo dye for measurement in step (c).

9. A reagent for the determination of LAP activity comprising a compound of formula (I) as defined in claim 1 and a chromogen reactive with the reaction product of that compound with LAP to produce a pigment or azo dye.

10. A reagent according to claim 9, which further contains an oxidizing agent.

11. A reagent according to claim 10, wherein the oxidizing agent is an oxidase.

12. A reagent for the determination of LAP activity comprising a compound of formula (I) as defined in claim 1, and a diazotising agent reactive with the reaction product of that compound with LAP.

13. A compound of formula (I) as defined in claim 1 or a salt thereof.

## Patentansprüche

1. Verfahren zur Bestimmung der Leucin-Aminopeptidase (LAP)-Aktivität in einer Probe, umfassend das Umsetzen des Enzyms mit einem Enzymsubstrat zur Gewinnung einer Produktverbindung, die mit Hilfe einer colorimetrischen Reaktion bestimmbar ist, **gekennzeichnet durch** die folgenden Schritte: (a) Umsetzung des Enzyms mit einem Enzymsubstrat der Formel (I): 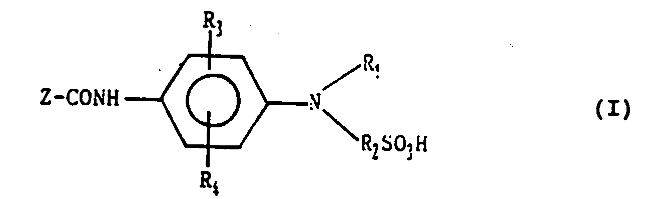 in der Z die Gruppe (CH₃)₂CHCH₂CH(NH₂)- darstellt, R₁ ein Wasserstoffatom, einen Alkylrest oder einen substituierten Alkylrest bedeutet, R₂ einen Alkylen- oder Hydroxyalkylenrest darstellt, R₃ und R₄ gleich oder verschieden sind und ein Wasserstoffatom, ein Halogenatom, eine Nitrogruppe, eine Hydroxylgruppe, eine Sulfogruppe, eine Carboxylgruppe, einen Alkylrest und einen Alkoxyrest oder ein Salz davon darstellen, zur Gewinnung einer Produktverbindung der Formel (II): 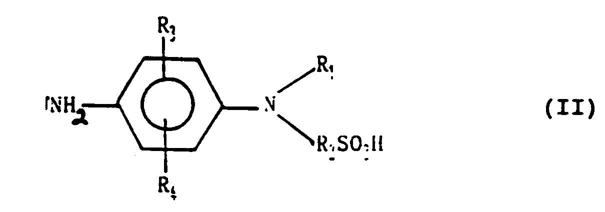 in der R₁, R₂, R₃ und R₄ wie vorstehend definiert sind; (b) Umsetzung der Verbindung der Formel (II) mit einem Chromogen zur Erzeugung eines Pigments oder Azofarbstoffes oder Umwandlung der Verbindung der Formel (II) in ein Diazoniumsalz und Umsetzung des Diazoniumsalzes mit einem Kupplungsmittels zur Erzeugung eines Azofarbstoffes; und (c) Messung der Absorption der farbigen Reaktionslösung.

2. Verfahren nach Anspruch 1, wobei in Schritt (b) die Umsetzung der Verbindung (II) mit dem Chromogen in Gegenwart eines Oxidationsmittels durchgeführt wird, wobei diese Umsetzung ein gefärbtes Produkt ergibt, das kein Azofarbstoff ist.

3. Verfahren nach Anspruch 2, wobei das Oxidationsmittel eine Oxidase ist.

4. Verfahren nach Anspruch 1, 2 oder 3, wobei in den Formeln (I) und (II) R₁ einen Sulfoalkylrest bedeutet.

5. Verfahren nach Anspruch 1, 2 oder 3, wobei das in Schritt (a) verwendete Enzymsubstrat L-Leucyl-p-N,N-disulfopropylaminoanilid ist.

6. Verfahren nach einem der Ansprüche 1 bis 5, wobei in Schritt (b) die Verbindung der Formel (II) mit einem Amin, Anilinderivat oder einem Phenol als Chromogen umgesetzt wird.

7. Verfahren nach Anspruch 6, wobei das Chromogen EMSE ist.

8. Verfahren nach Anspruch 1, 4 oder 5, wobei in Schritt (b) die Verbindung der Formel (II) mit Zimtaldehyd umgesetzt wird, so daß ein Azofarbstoff für die Messung in Schritt (c) erzeugt wird.

9. Reagens für die Bestimmung der LAP-Aktivität, umfassend eine Verbindung der Formel (I) gemäß der Definition in Anspruch 1 und ein Chromogen, das mit dem Reaktionsprodukt dieser Verbindung mit LAP unter Erzeugung eines Pigments oder Azofarbstoffs reaktiv ist.

10. Reagens nach Anspruch 9, das weiterhin ein Oxidationsmittel enthält.

11. Reagens nach Anspruch 10, wobei das Oxidationsmittel eine Oxidase ist.

12. Reagens zur Bestimmung der LAP-Aktivität, umfassend eine Verbindung der Formel (I) gemäß der Definition in Anspruch 1 und ein Diazotierungsmittel, das mit dem Reaktionsprodukt dieser Verbindung mit LAP reaktiv ist.

13. Verbindung nach Anspruch 1 gemäß der Definition in Anspruch 1 oder ein Salz davon.

## Revendications

1. Procédé de dosage de l'activité de leucine aminopeptidase (LAP) dans un échantillon, lequel procédé comporte la réaction de l'enzyme avec un substrat pour cette enzyme, réaction qui donne comme produit un composé dosable par colorimétrie, ledit procédé étant caractérisé par les étapes consistant à :
(a) faire réagir l'enzyme avec un substrat de formule (I) : 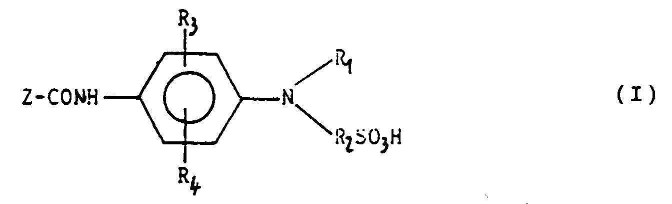 dans laquelle Z représente (CH₃)₂CHCH₂CH(NH₂)-, R₁ représente un atome d'hydrogène ou un groupe alkyle ou alkyle substitué, R₂ représente un groupe alkylène ou hydroxyalkylène, et R₃ et R₄, identiques ou différents, représentent chacun un atome d'hydrogène ou d'halogène ou un groupe nitro, hydroxyle, sulfo, carboxyle, alkyle ou alcoxy,
ou avec un sel d'un tel composé, de façon à obtenir comme produit un composé de formule (II) : 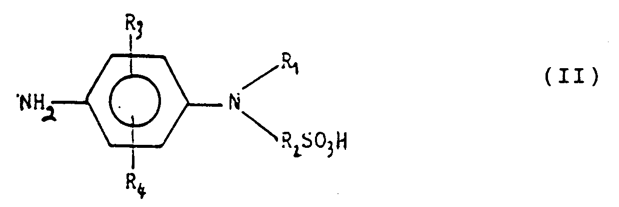 dans laquelle R₁, R₂, R₃ et R₄ sont tels que définis ci-dessus ;
(b) faire réagir le composé de formule (II) avec un chromogène de façon à former un pigment ou un colorant azoïque, ou convertir le composé de formule (II) en un sel de diazonium et faire réagir ce sel de diazonium avec un copulant de façon à former un colorant azoïque ; et
(c) mesurer l'absorption de la solution réactionnelle colorée.

2. Procédé conforme à la revendication 1, dans lequel, dans l'étape (b), on réalise la réaction du composé (II) avec le chromogène en présence d'un agent oxydant, cette réaction donnant un produit coloré autre qu'un colorant azoïque.

3. Procédé conforme à la revendication 2, dans lequel ledit agent oxydant est une oxydase.

4. Procédé conforme à la revendication 1, 2 ou 3, dons lequel, dans les formules (I) et (II), R₁ représente un groupe sulfoalkyle.

5. Procédé conforme à la revendication 1, 2 ou 3, dans lequel le substrat utilisé pour l'enzyme dans l'étape (a) est le L-leucyl-p-N,N-disulfopropylaminoanilide.

6. Procédé conforme à l'une quelconque des revendications 1 à 5, dans lequel, dans l'étape (b), on fait réagir le composé de formule (II) avec une amine, un dérivé de l'aniline ou un phénol, en tant que ledit chromogène.

7. Procédé conforme à la revendication 6, dans lequel le chromogène est l'EMSE.

8. Procédé conforme à la revendication 1, 4 ou 5, dans lequel, dans l'étape (b), on fait réagir le composé de formule (II) avec du cinnamaldéhyde pour obtenir un colorant azoïque pour la mesure effectuée dans l'étape (c).

9. Réactif pour le dosage de l'activité de LAP, comprenant un composé de formule (I) tel que défini dans la revendication 1 et un chromogène qui réagit avec le produit de réaction de ce composé avec la LAP pour donner un pigment ou un colorant azoïque.

10. Réactif conforme à la revendication 9, qui contient en outre un agent oxydant.

11. Réactif conforme à la revendication 10, dans lequel l'agent oxydant est une oxydase.

12. Réactif pour le dosage de l'activité de LAP, comprenant un composé de formule (I) tel que défini dans la revendication 1 et un agent de diazotation qui réagit avec le produit de réaction de ce composé avec la LAP.

13. Composé de formule (I) tel que défini dans la revendication 1, ou sel d'un tel composé.
